# EUROPEAN PATENT SPECIFICATION

(11) **EP 0 642 322 B1**
(45) Date of publication and mention of the grant of the patent: **06.12.1995**
(21) Application number: 93912732.0
(22) Date of filing: 21.05.1993
(51) Int. Cl.: A61B 17/39, A61M 1/00

(54) **Apparatus for cosmetical treatment of the human body through removal of adipose masses**
Vorrichtung zur kosmetischen Behandlung eines menschlichen Körpers durch Entfernung von Fettgewebe
Appareil pour le traitement cosmétique du corps humain par élimination des masses adipeuses

(30) Priority: 27.05.1992 IT TO920454
(43) Date of publication of application: 15.03.1995
(73) Proprietor: L.I.C.A. di ROSSO & C. S.n.c., I-10040 Caselette (Torino) (IT)
(72) Inventor: ROSSO, Luciano, I-10040 Caselette (IT)
(74) Representative: Buzzi, Franco
(86) International application number: EP9301258
(87) International publication number: WO9324066

(56) References cited:
- EP-A- 0 331 313
- WO-A-91/12774
- DE-B- 1 143 937

## Description

### Technical field

The present invention is related to cosmetical treatments of the human body, and is more particularly concerned with an apparatus for the removal of masses of adipose or fatty tissue according to the preamble of claim 1.

### Background art

A system for the removal of masses of adipose or fatty tissue, comprising a fluidizing step by means of the combined effect of infiltration of a diluent solution and application of a fluidizing perturbation within the adipose tissue, and a step of extraction of the fluidized adipose tissue, is disclosed, for example, in Italian magazine "Salve", No. 2 February 1992, RCS Rizzoli Periodici. This known system, the final purpose of which is to produce (according to EP-A-0418979) autologous collagene from the human adipose tissue, contemplates for removal of the adipose tissue firstly the surgical cut of the body area to be treated, subsequently the introduction through the cut of a cannula for the infiltration of an anaesthetic and diluting solution, and then the insertion of a probe-lancet provided with a piezoelectric transducer for the generation of ultra-sounds. The ultra-sounds produce high frequency mechanical vibrations, and thus shock waves which impact the molecules of the endo-cellular substances thus causing the crushing of the adipocites. A cellular emulsion is then produced, constituted by the contents of the fatty cells fragments, by inter-cellular substances and by the preliminarly injected solution. These substances are subsequently removed by means of a "squeezing" action, whereby the liquified fat is drawn outside the surgical cuts by squeezing, energetically massaging the treated part by means of a roller device.

This system has a series of drawbacks: firstly, it involves a real surgical operation, with the need of subsequent suture of the cuts and consequent dressings, as well as stay in hospital. Secondly, for these very reasons, the treatment can be only performed locally, and repetition thereof in different areas of the body can be made only after a certain period of time. Last but not least, the treatment requires the intervention of a specialized surgeon and of a medical staff.

EP-A-0331313 and WO-A-91/12774 are also directed to soft tissue aspiration, employing in the first case ultrasonic vibration and in the second case laser energy. Namely, EP-A-0331313 relates to an apparatus as defined in the preamble of claim 1. The fluidizing perturbation is carried out by means of an ultrasonic aspirating probe. The probe is ultrasonically vibrated at high frequencies and low amplitudes creating localized tissue separation and frictional heat. This heat causes the fatty tissue surrounding the probe to melt. An irrigating fluid serves to emulsify the melted and separated fat, which is then aspirated.

The results obtained by this known apparatus are reported to be rather unsatisfactory.

### Disclosure of the invention

The object of the present invention is to overcome the above drawbacks, and this object is achieved by virtue of an apparatus of the above-referenced type, the main feature of which is recited in the charaterising portion of claim 1.

Due to this idea of solution, the apparatus according to the invention enables the treatment to be carried out without any surgical intervention and in a more complete way, intervening consecutively and in rapid succession on the different areas of the bodies requiring the treatment. The emission of electro-magnetic waves through the adipose tissue further enables the obtention of a more efficient and more rapid fluidizing effect, deriving from the controlled and localized heating of the area under treatment. This effect is particularly appreciable if, according to a first embodiment of the invention, the electro-magnetic waves are radio-waves, in a frequency range conveniently comprised between 0,3 and 2 MHz.

According to an alternative embodiment, the frequency of the electro-magnetic waves is within the low-frequency range, conveniently comprised between 30 and 1000 Hz. In this case a less remarkable perturbing effect is obtained, nevertheless such as to effectively stimulate the tissues to be removed.

### Brief description of drawings

The invention will now be described in detail with reference to the annexed drawings, provided purely by way of non-limiting example, wherein:

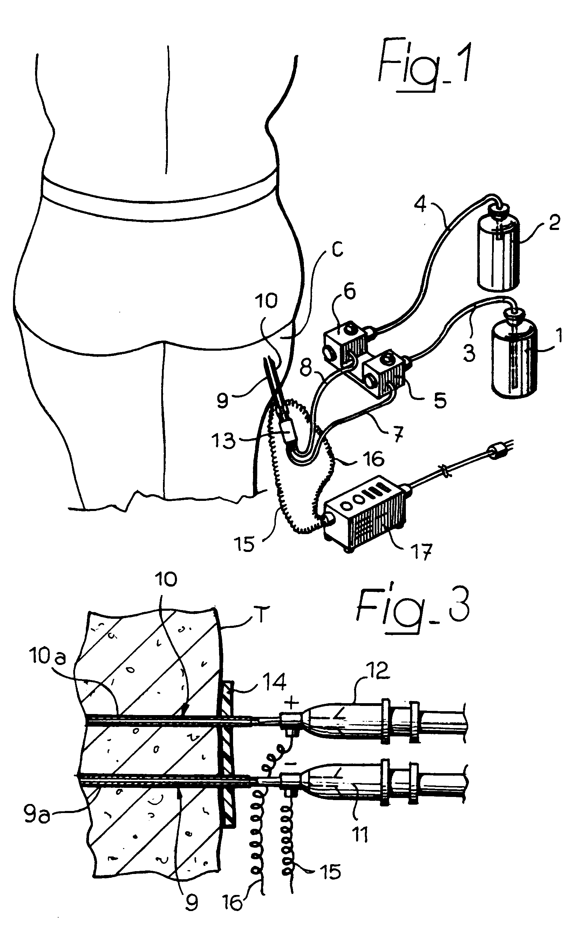

- figure 1 is a diagrammatic perspective view of an apparatus for cosmetical treatment according to the invention,

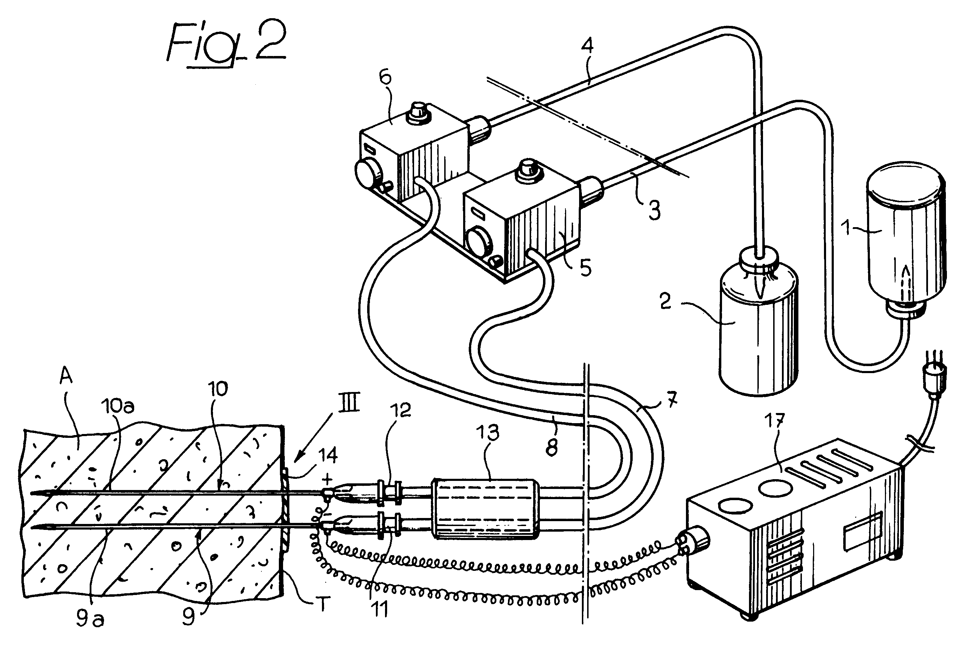

- figure 2 is a view similar to figure 1 but in an enlarged scale, and
- figure 3 is partially sectioned view of a part of the apparatus.

### Best mode for carrying out the invention

Referring to the drawings, references numerals 1 and 2 respectively designate a first closed vessel containing a fluidising liquid (for example distilled water or a physiological solution possibly with diluent and/or anaesthetic components), and a second closed vessel intended to receive therein the adipose tissues removed by an area of the human body C. The vessels 1 and 2 are connected, through respective hoses 3 and 4, to a delivery peristaltic pump 5 and to a suction peristaltic pump 6, respectively, both provided in a known way with speed control. The two pumps 5 and 6, which can be replaced by pneumatic or other type pumps, are in turn connected by means of respective tubes 7 and 8 to a pair of thin tubular perforator needles 9 and 10, parallel to each other and provided with respective pipe fittings 11 and 12 carried, in a side by side configuration, by a handle 13. The handle 13 with the perforator needles 9 and 10 can be of disposable type.

The two perforators 9 and 10, which conveniently cross a centering and guide member 14, are made of an electrically conductive material and are lined, at least for part of their length, by an electrically and thermally insulating covering 9a, 10a,respectively, for instance made of silicone or the like.

Adjacent to the pipe fittings 11 and 12, the perforator needles 9 and 10 are electrically connected, externally or internally, by means of respective conductor wires 15, 16, to an electro-magnetic wave generator 17. The perforator needles 9 and 10 thus also act as electrodes for the emission, during operation, of the electro-magnetic waves produced by the generator 17.

According to a first embodiment of the invention, the generator 17 includes an oscillator circuit, not shown but within the knowledge of the man skilled in the art, for the generation of radio waves, preferably in a frequency range comprised between 0,3 and 2 MHz (medium and intermediate waves).

According to another embodiment, the generator 17 produces, also in a way known per se, low frequency electro-magnetic waves, conveniently comprised in a range between 30 and 1000 Hz.

In a further embodiment, the generator 17 is designed so as to selectively generate electro-magnetic waves comprised between 30 Hz and 2 MHz, so as to cover both the low frequency range and the radio frequency range, under discretion of the operator and even in succession.

In use, the perforator needles 9 and 10 are introduced, through the skin T of the patient, within the adipose tissue A to be treated. The phase of introduction, which simply involves two small perforations, is facilitated by the centering and guide member 14, which during this operation can be maintained against the skin T.

Thereafter, after checking that no interference with veins or arteries has occurred, the peristaltic pump 5 is operated, so as to feed the diluent solution contained in the vessel 1 (normally without any need of pre-heating) into the adipose tissue A. Simultaneously the generator 17 is activated, which through the electrodes constituted by the perforators 9 and 10 emits electro-magnetic waves at low frequency, or radio-frequency, or in succession firstly at low frequency and subsequently radio frequency, within the adipose tissue A. As explained in the above, the low frequency is normally comprised between 30 and 1000 Hz and the radio frequency is in the range between 0,3 and 2 MHz.

The emission capacity of the generator 17 can be adjusted, and is variable for instance between 0 and 100 Watt.

The emission of the electro-magnetic waves through the adipose tissue A, combined with the action of the diluent solution delivered by the pump 5, carries out an effective fluidising action of the adipose tissue which can then be removed by suction under the action of the pump 6, through the perforator 10, and collected within the vessel 2.

Operation of the generator 17 at low frequency performs a perturbating effect of stimulation of the tissue to be removed, while the radio-frequency accomplishes a more important and rapid fluidizing effect.

The coatings 9a and 10a of the perforator needles 9 and 10 permit to efficiently insulate, both electrically and thermally, the skin T which, as it is known, is the only sensitive part in the crossing area of the perforators 9 and 10.

The possibility of adjusting the peristaltic pumps 5 and 6 and the electro-magnetic wave generator 17 enables a wide adjustment of the treatment, which can be dosed as a function of the specific characteristics of the patient.

Naturally, the details of construction and the embodiments of the apparatus can be widely varied with respect to what has been disclosed and illustrated, without thereby departing from the scope of the present invention.

Thus, for example, according to another embodiment not shown in the drawings, only a single perforator instead of two can be employed, connected by means of a two-way connector to both tubes 7 and 8, and thus to both pumps 5, 6 and vessels 1, 2. In this case, the second electrode will be constituted by an outer plate to be placed in contact with the body C and of course also connected to the generator 17, and the fluidising and extraction steps of the adipose tissue A will be carried out in a cyclically alternated way, operating in succession firstly one and then the other of the pumps 5, 6.

Moreover, in this case, a single reversible peristaltic (or of a different type) pump can be used, instead of two separate pumps.

According to a further embodiment, a plurality of needles can be employed, arranged according to one or more formations and carried by the handle 13.

Lastly, it is to be pointed out that the electro-magnetic wave generator 17 can be operated during the fluidising phase either continuosly, or so as to produce pulses or pulse trains of variable length, separated by pauses, also variable, adjusted manually or by means of a programmable micro-processor incorporated in the apparatus and within the knowledge of the man skilled in the art.

## Claims

1. Apparatus for the cosmetical treatment of the human body (C) through removal of adipose tissue masses (A), comprising means for infiltrating a diluent solution and means for applying a fluidizing perturbation within the adipose tissue (A), and means for the extraction of the fluidized adipose tissue (A), characterized in that the means for applying the fluidizing perturbation comprise a generator (17) of electro-magnetic waves in a frequency range comprised between 30 Hz and 2 MHz, and a pair of electrodes, to at least one of which is directly associated a tubular perforator (9, 10) for the infiltration of the diluent solution and/or for extraction of the fluidized adipose tissue.

2. Apparatus according to claim 1, characterized in that to the said at least one tubular perforator (9, 10) at least one hose (7, 8) is connected, to which a peristaltic pump (5, 6) is operatively associated.

3. Apparatus according to claim 2, characterized in that a closed vessel (1, 2) is connected to the said at least one hose (7, 8).

4. Apparatus according to claim 1, characterized in that it comprises a pair of tubular perforators (9, 10) to each of which a respective electrode is associated.

5. Apparatus according to claim 1 or claim 4, characterized in that the or each tubular perforator (9, 10) has at least an outer portion covered by a thermal and electric insulating material (9a, 10a).

6. Apparatus according to claim 2, characterized in that the or each peristaltic pump (5, 6) has a variable speed.

7. Apparatus according to claim 1, characterized in that the electro-magnetic wave generator (17) is of variable capacity.

8. Apparatus according to claim 1, characterized in that the electro-magnetic wave generator comprises a low frequency oscillator in a frequency range comprised between 30 and 1000 Hz.

9. Apparatus according to claim 1 or claim 8, characterized in that the electro-magnetic wave generator comprises a radio-frequency oscillator in a frequency range comprised between 0,3 and 2 MHz.

## Patentansprüche

1. Gerät zur kosmetischen Behandlung des menschlichen Körpers (C) durch Entfernung von adipösen Gewebsmassen (A), welches Mittel zur Einführung einer Verdünnungslösung und Mittel zur Anwendung einer verflüssigenden Massage innerhalb des adipösen Gewebes (A) und Mittel zur Extraktion des verflüssigten adipösen Gewebes (A) umfasst, dadurch gekennzeichnet, dass das Mittel zur Anwendung der verflüssigenden Massage einen Generator (17) für elektromagnetische Wellen in einem Frequenzbereich von 30 Hz und 2 MHz umfasst, und ein Paar Elektroden, von denen mindestens eine direkt mit einem tubulären Perforator (9, 10) zur Einführung der Verdünnungslösung und/oder zur Extraktion des verflüssigten adipösen Gewebes verbunden ist.

2. Gerät gemäss Anspruch 1, dadurch gekennzeichnet, dass genanntes Gerät mit mindestens einem tubulären Perforator (9, 10) und mit mindestens einem Schlauch (7, 8) verbunden ist, mit dem eine peristaltische Pumpe (5, 6) funktionsfähig verbunden ist.

3. Gerät gemäss Anspruch 2, dadurch gekennzeichnet, dass ein geschlossenes Gefäss (1, 2) mit dem genannten Gerät über mindestens einen Schlauch (7, 8) verbunden ist.

4. Gerät gemäss Anspruch 1, dadurch gekennzeichnet, dass es ein Paar tubulärer Perforatoren (9, 10) umfasst, die mit jeweils einer Elektrode verbunden sind.

5. Gerät gemäss Anspruch 1 oder Anspruch 4, dadurch gekennzeichnet, dass der oder jeder tubuläre Perforator (9, 10) mindestens einen äusseren Teil besitzt, der von einem thermisch und elektrisch isolierenden Material (9a, 10a) umgeben ist.

6. Gerät gemäss Anspruch 2, dadurch gekennzeichnet, dass die oder jede peristaltische Pumpe (5, 6) eine variable Geschwindigkeit besitzt.

7. Gerät gemäss Anspruch 1, dadurch gekennzeichnet, dass der elektromagnetische Wellengenerator (17) eine variable Kapazität besitzt.

8. Gerät gemäss Anspruch 1, dadurch gekennzeichnet, dass der elektromagnetische Wellengenerator einen Niederfrequenzoszillator in einem Frequenzbereich besitzt, der zwischen 30 und 1000 Hz umfasst.

9. Gerät gemäss Anspruch 1, dadurch gekennzeichnet, dass der elektromagnetische Wellengenerator einen Radiofrequenzoszillator in einem Frequenzbereich von 0,3 und 2 MHz umfasst.

## Revendications

1. Appareil pour le traitement esthétique du corps humain (C) par retrait des masses de tissu adipeux (A), comprenant des moyens pour faire pénétrer une solution de diluant et des moyens pour appliquer une perturbation fluidisante dans le tissu adipeux (A), et des moyens pour l'extraction du tissu adipeux (A) fluidisé, caractérisé en ce que les moyens pour appliquer la perturbation fluidisante comprennent un générateur (17) d'ondes électromagnétiques dans un domaine de fréquences compris entre 30 Hz et 2 MHz, et une paire d'électrodes à au moins l'une desquelles est associé directement un perforateur tubulaire (9, 10) pour la pénétration de la solution de diluant et/ou pour l'extraction du tissu adipeux fluidisé.

2. Appareil selon la revendication 1, caractérisé en ce que, au(x)dit(s) perforateur(s) tubulaire(s) (9, 10) et relié au moins un tuyau souple (7, 8) auquel est associée de manière active une pompe péristaltique (5, 6).

3. Appareil selon la revendication 2, caractérisé en ce qu'un récipient fermé (1, 2) est relié au(x)dit(s) tuyau(x) souple(s) (7, 8).

4. Appareil selon la revendication 1, caractérisé en ce qu'il comprend une paire de perforateurs tubulaires (9, 10) à chacun desquels est associée une électrode respective.

5. Appareil selon la revendication 1 ou la revendication 4, caractérisé en ce que le ou chaque perforateur tubulaire (9, 10) a au moins une partie externe recouverte par un matériau thermiquement et électriquement isolant (9a, 10a).

6. Appareil selon la revendication 2, caractérisé en ce que la ou chaque pompe péristaltique (5, 6) a une vitesse variable.

7. Appareil selon la revendication 1, caractérisé en ce que le générateur d'ondes électromagnétiques (17) est de capacité variable.

8. Appareil selon la revendication 1, caractérisé en ce que le générateur d'ondes électromagnétiques comprend un oscillateur basse fréquence dans un domaine de fréquences compris entre 30 et 1 000 Hz.

9. Appareil selon la revendication 1 ou la revendication 8, caractérisé en ce que le générateur d'ondes électromagnétiques comprend un oscillateur radio-fréquence dans un domaine de fréquences compris entre 0,3 et 2 MHz.
